# NOUVEAU FASCICULE DE BREVET EUROPEEN

(11) **EP 1 008 602 B2**
(45) Date de publication et mention de la décision concernant l'opposition: **21.11.2018**
(45) Mention de la délivrance du brevet: 23.04.2003
(21) Numéro de dépôt: 99403095.5
(22) Date de dépôt: 09.12.1999
(51) Int. Cl.: C07H 3/06, C07C 31/26, A61K 9/20, A23G 3/00, A23L 1/236

(54) **Sorbitol pulv?rulent et son proc?d? de pr?paration**
Pulverisierte-Sorbitol und ihr Verfahren zur Herstellung
Powdered sorbitol and its process for preparation

(30) Priorité: 11.12.1998 FR 9815681
(43) Date de publication de la demande: 14.06.2000
(73) Titulaire: Roquette Frères, 62136 Lestrem (FR)
(72) Inventeur: Moraly, Franck, 62136 Lestrem (FR); Labergerie, Erik, 59280 Armentieres (FR); Lis, José, 59253 La Gorgue (FR); Lefevre, Philippe, 59400 Merville (FR); Bouvier, Frédéric, 59000 Lille (FR)
(74) Mandataire: Cabinet Plasseraud

(56) Documents cités:
- EP-A- 0 032 288
- EP-A1- 0 645 096
- EP-A2- 0 380 219
- FR-A- 2 622 190
- GB-A- 2 046 743
- CERTIFICAT D'ANALYSE MERCK DU "3583 KARION PULVER (SORBIT)", DATE DU 13.07.1994.
- CERTIFICAT D'ANALYSE DU "1.03583.9050 SORBITOL POWDER PH EUR.BP.NF, E420" AVEC COMME DATE D'EXAMEN LE 04.08.1998
- TRANSCRIPTION D'UN E-MAIL MERCK KGAA DATE DU 13.01.2004
- CATALOGUE "SORBITOL MERCK AND ITS APPLICATIONS" DATé DU 08.06.1998
- DÉCLARATION DE PASCALE DE MEUTER DATÉE DU 06.01.2004
- ECHANGE DE FAXES DATéS DU 17/21.03.2003 DEMANDANT DES INFORMATIONS SUR LA MACHINE UTILISéE POUR TESTER LA FRIABILITé DANS EP0645096
- "ISO-STANDARD SIEVING METHODS (2591)", PUBLIé EN 1988
- DÉCLARATION DATÉE DU 02.04.2002 DE LIESBETH MARIA FERNANDE MEEUS, EMPLOYÉE DE CERESTAR
- LETTRE DE PROPRIÉTAIRE DU BREVET SOUMISE LORS DE LA PROCÉDURE D'EXAMEN DU BREVET INCRIMINÉ.
- BON DE COMMANDE ET DE LIVRAISON CONCÈRNANT LE PRODUIT 3583 KARION PULVER (SORBIT)

## Description

La présente invention a pour objet un sorbitol pulvérulent de faible hygroscopicité et de surface spécifique élevée, présentant par ailleurs une faible densité, une granulométrie particulière et une excellente aptitude à l'écoulement.

L'invention concerne également un sorbitol pulvérulent dont les propriétés techniques d'utilisation en compression directe sont améliorées, ainsi qu'un procédé pour sa préparation.

Le sorbitol est un hexitol principalement utilisé dans les domaines d'applications alimentaire et pharmaceutique à titre d'agent sucrant, mais également pour sa caloricité réduite et son acariogénicité.

Le sorbitol pulvérulent, tout comme les autres polyols pulvérulents tels le xylitol ou le mannitol, est quant à lui d'usage courant comme excipient pharmaceutique, comme édulcorant et agent de texture en industrie alimentaire, et comme support d'additif dans tous types d'industries. Il est cependant meilleur excipient que le xylitol et le mannitol, notamment en compression, en raison de son aptitude particulière à cristalliser sous forme de cristaux en aiguilles, directement compressibles.

En général, pour disposer d'un sorbitol cristallisé de grande résistance en compression, on s'efforce de fabriquer un sorbitol de forme cristalline γ (les formes α et β sont particulièrement instables) en travaillant une solution sursaturée en sorbitol dont la forme γ représente au moins 90 %. Cependant, même lorsqu'il est cristallisé sous cette forme γ la plus stable, le sorbitol pulvérulent obtenu classiquement présente un certain nombre d'inconvénients dont celui d'être très hygroscopique.

Cette hygroscopicité élevée conduit à rendre l'écoulement du sorbitol pulvérulent difficile, voire impossible, dès lors qu'une reprise en eau est intervenue. Son utilisation en compression directe s'en trouve alors limitée, nécessitant par exemple de surmonter de sérieuses difficultés de remplissage des presses pour la fabrication de tablettes ou de comprimés.

Pour éviter ce problème d'écoulement du sorbitol pulvérulent, il a été préconisé de préparer un sorbitol de densité faible et de granulométrie plus grossière, comme décrit dans le brevet FR 1 506 334.

Cependant, il est établi que plus la densité apparente d'un sorbitol pulvérulent est faible, plus celui-ci devient friable, c'est-à-dire sensible à une altération de sa granulométrie par action mécanique. En outre, les temps de dissolution de ce produit pulvérulent de granulométrie grossière sont en général trop longs et donc inadaptés.

Enfin, si l'aptitude à l'écoulement est partiellement améliorée par la mise en oeuvre de particules d'une telle granulométrie, le caractère hygroscopique résiduel encore trop élevé rend dans tous les cas l'utilisation de ce sorbitol pulvérulent rédhibitoire lorsqu'il est associé à des ingrédients ou à des additifs très sensibles à l'eau.

Il est également établi que la capacité à fixer des quantités importantes d'additif est directement fonction de la surface spécifique desdites particules. Les capacités d'absorption du sorbitol pulvérulent sont ainsi d'autant plus importantes que sa surface spécifique est élevée. Cependant, il est connu que la surface spécifique des cristaux denses de γ sorbitol du commerce est très faible. Ainsi, pour une taille de particules comprise entre 500 et 1000 µm, elle est très faible, i.e. au plus égale à 0,7 m²/g.

Dans le but de préparer un sorbitol pulvérulent présentant une meilleure granulométrie, une bonne aptitude à l'écoulement et satisfaisant aux conditions de compressibilité et de friabilité souhaitées, la demande de brevet FR 2 622 190 décrit un sorbitol poudre renfermant des particules d'un diamètre moyen compris entre 300 et 500 µm. Mais la densité apparente élevée et la surface spécifique relativement faible desdites particules, de l'ordre de 0,9 à 1,2 m²/ g, ne sont en fait pas significativement modifiées par le procédé de fabrication mis en oeuvre, de sorte que le sorbitol pulvérulent ainsi obtenu conserve le même facteur d'absorption d'humidité et la même solubilité dans l'eau que la poudre de sorbitol de départ.

Le brevet EP 32 288 décrit un polymorphe de sorbitol y de structure cristalline désintégrée et lâche, présentant une hygroscopicité améliorée et des propriétés de compression satisfaisantes. Cependant, ces propriétés particulières ne sont relatives qu'à une coupe granulométrique comprise entre 250 et 841 µm (i.e. 20/60 mesh), dont la surface spécifique est inférieure, en tout état de cause, à 2 m²/g.

De tout ce qui précède, il ressort qu'il existe un besoin non satisfait de disposer d'un sorbitol pulvérulent qui présente à la fois les avantages, le plus souvent incompatibles, de faible hygroscopicité d'une part et de surface spécifique élevée d'autre part ou de faible densité apparente d'une part et de faible friabilité d'autre part et ce, pour une taille de particules relativement faible.

La société Demanderesse a donc eu le mérite de concilier tous ces objectifs réputés jusqu'alors inconciliables en imaginant et élaborant, au prix de nombreuses recherches, un nouveau sorbitol pulvérulent.

Le sorbitol pulvérulent conforme à l'invention est ainsi tout d'abord caractérisé en ce qu'il présente :
- une valeur d'hygroscopicité, déterminée selon un test A, inférieure à 2 %, de préférence inférieure à 1,7 %,
- une surface spécifique, déterminée selon BET, au moins égale à 2 m²/g, de préférence au moins égale à 2,2 m²/g.

De préférence, son hygroscopicité, déterminée selon le test A, est comprise entre 0,5 et 1,6 %, préférentiellement comprise entre 0,9 et 1,4 %.

Le test A consiste à établir la courbe d'isotherme de sorption d'eau à 20°C exprimant le pourcentage de reprise en eau d'un produit pulvérulent préalablement déshydraté que l'on place dans une atmosphère de teneur en humidité relative variable et à une température de 20°C. La détermination de l'hygroscopicité du produit pulvérulent sera alors le pourcentage de reprise en eau, à 60 % d'humidité relative à l'équilibre (ou 60 % E.R.H. pour Equilibrium Relative Humidity).

On détermine la surface spécifique sur l'ensemble de la distribution granulométrique du sorbitol pulvérulent grâce à un analyseur de surface spécifique Quantachrome basé sur un test d'absorption de l'azote sur la surface du produit soumis à l'analyse, en suivant la technique décrite dans l'article BET Surface Area by Nitrogen Absorption de S. BRUNAUER et al. (Journal of American Chemical Society, 60, 309, 1938).

Il est particulièrement surprenant qu'un sorbitol pulvérulent puisse présenter conjointement une surface spécifique au moins égale à 2 m²/g, de préférence au moins égale à 2,2 m²/g et une hygroscopicité inférieure à 2 %, de préférence inférieure à 1,7 %. En effet, il est admis très classiquement que l'hygroscopicité d'un produit pulvérulent augmente avec sa surface spécifique, i.e. sa surface exposée au milieu contenant de la vapeur d'eau.

Or, le sorbitol pulvérulent conforme à l'invention présente une surface spécifique élevée, caractéristique d'un produit granulé, avec pourtant une faible hygroscopicité, caractéristique d'un produit cristallisé sous une forme cristalline stable.

A titre d'exemples, le sorbitol commercialisé par la société MERCK sous l'appellation SORBITOL grade L présente une hygroscopicité de 2,4 % % à 60 % d'ERH selon le test A, pour une surface spécifique selon BET de 1,55 m²/g, et le sorbitol commercialisé par la société Demanderesse sous la marque NEOSORB® P 60 W, présente une hygroscopicité, dans les mêmes conditions de mesure, d'une valeur de 1,53 % pour une surface spécifique inférieure à 1m²/g.

De façon surprenante et inattendue et. contrairement à ce que l'on attendait, le sorbitol pulvérulent conforme à l'invention présente une hygroscopicité remarquablement plus faible que ce qui est classiquement décrit pour les sorbitol pulvérulents du commerce présentant les surfaces spécifiques les plus élevées. Il en résulte que le sorbitol pulvérulent conforme à l'invention possède de bien meilleures propriétés, notamment comme support d'additif, qu'un sorbitol pulvérulent standard. Celles-ci sont dues, au moins en partie, à la surface spécifique particulièrement élevée de ce produit.

A ce titre, la société Demanderesse considère comme nouveau un sorbitol pulvérulent caractérisé en ce qu'il présente une surface spécifique selon BET supérieure a 2,5 m²/g, de préférence comprise entre 2,6 et 4 m²/g, et plus préférentiellement encore comprise entre 2,6 et 3,5 m²/g.

Le sorbitol pulvérulent conforme à l'invention peut également être caractérisé par sa densité apparente, ses propriétés en compression directe et sa friabilité.

La détermination de la densité apparente est réalisée par l'utilisation d'un appareil commercialisé par la société HOSOKAWA sous la marque POWDER TESTER en appliquant la méthode recommandée pour mesurer une densité apparente.

Dans ces conditions, le sorbitol pulvérulent conforme à l'invention présente une densité apparente faible, c'est à dire comprise entre 0,35 et 0,65 g/ml, de préférence comprise entre 0,4 et 0,6 g/ml.

La compressibilité du sorbitol pulvérulent est déterminée selon le test B suivant, décrit dans le brevet EP 220.103 dont la Société Demanderesse est titulaire. Ce test B consiste à mesurer la force, exprimée en Newton, qui est représentative de la compressibilité du sorbitol pulvérulent étudié. Cette force traduit donc ici la résistance à l'écrasement d'un comprimé qui est cylindrique à faces convexes (rayon de courbure de 14 mm), d'un diamètre de 13 mm, d'une épaisseur de 6 mm et d'un poids de 0,647 g, soit d'une masse volumique apparente de 1,1 g/ml.

La compressibilité du sorbitol pulvérulent conforme à l'invention est déterminée à une valeur généralement comprise entre 100 et 150 N, plus particulièrement à une valeur comprise entre 120 et 140 N.

La friabilité du sorbitol pulvérulent conforme à l'invention est quant à elle déterminée selon un test C, décrit dans le brevet EP 645 096 dont la société Demanderesse est titulaire.

Ce test C consiste à soumettre 15 g de sorbitol pulvérulent à tester, ayant une taille de particule comprise entre 100 et 200 µm, pendant 15 minutes, à l'action mécanique d'un friabilimètre ERWEKA TAP de la société HERWE-KA (6056 Heusenstamm - DE), en rotation à une vitesse uniforme de 25 tours par minute, dans lequel 5 billes d'acier identiques avec un diamètre de 17 mm et un poids de 18,87 g ont été introduites, la friabilité selon ce test étant le pourcentage en poids de poudre non retenue après le test sur un tamis ayant une largeur de maille de 100 µm.

La friabilité du sorbitol pulvérulent conforme à l'invention présente une valeur généralement comprise entre 10 et 50 %, de préférence comprise entre 20 et 40 %.

Cette valeur de friabilité est d'autant plus remarquable que le sorbitol pulvérulent conforme à l'invention présente une faible densité. En effet, il est classiquement admis qu'un sorbitol pulvérulent sera d'autant moins friable que sa densité et sa compressibilité seront plus élevées.

De par cette compressibilité élevée, la résistance mécanique des comprimés obtenus avec ledit sorbitol pulvérulent est en effet particulièrement élevée, comparativement à celle des comprimés obtenus avec les produits du commerce. A titre d'exemple, les qualités de sorbitol poudre commercialisées par la société DHW RODLEBEN, de densité égale à 0,6 g/ml, présentent une compressibilité, déterminée selon le test B, voisine de 100 N.

La faible densité du sorbitol pulvérulent conforme à l'invention et son caractère de résistance mécanique élevée permettent de réduire avantageusement la matière mise en oeuvre pour la fabrication des comprimés ou de tablettes, diminuant par la même significativement les coûts de production.

S'agissant de la friabilité, de manière surprenante et inattendue, et contrairement à ce qui est communément admis, le sorbitol pulvérulent conforme à l'invention ne vérifie pas la règle selon laquelle plus la densité apparente d'un sorbitol pulvérulent est faible, plus sa friabilité est élevée.

Ces propriétés améliorées de compressibilité et de friabilité, combinées avec les caractères de faible hygroscopicité et de surface spécifique élevée ont notamment pour conséquence de rendre le sorbitol pulvérulent conforme à l'invention particulièrement bien adapté aux applications alimentaire, pharmaceutique ou autres, nécessitant la fixation d'importantes quantités d'additifs. A ce titre, ses capacités d'absorption nettement supérieures à celles des sorbitols compressibles connus classiquement lui permettent par ailleurs de supporter des additifs particulièrement solubles dans l'eau tels les vitamines, les colorants, les édulcorants intenses.

Par ailleurs, on peut également caractériser le sorbitol pulvérulent conforme à l'invention par son diamètre moyen, son uniformité de distribution granulométrique, et son aptitude à l'écoulement, ces propriétés convenant particulièrement auxdites applications en compression. Ainsi, le -sorbitol pulvérulent conforme à l'invention présente généralement un diamètre moyen compris entre 150 et 250 µm, et une distribution granulométrique comprise entre 60 et 500 µm. Ces valeurs sont déterminées sur un granulomètre Laser LS de marque COULTER®.

Par ailleurs, il faut souligner que les avantages non négligeables de l'utilisation du sorbitol pulvérulent conforme à l'invention cités ci-avant constituent un ensemble de propriétés que les produits de l'art antérieur, ne possèdent jamais simultanément. Parmi ces avantages, on peut citer son aptitude à l'écoulement.

Cette aptitude à l'écoulement est évaluée en utilisant l'appareil POWDER TESTER commercialisé par la société HOSOKAWA. Cet appareil permet de mesurer, dans des conditions standardisées et reproductibles, l'aptitude à l'écoulement d'une poudre et de calculer une note d'écoulement, encore appelée indice de Carr.

Le sorbitol pulvérulent conforme à l'invention présente une note d'écoulement excellente, généralement d'au moins 70, de préférence comprise entre 70 et 90, et plus préférentiellement comprise entre 70 et 80. Cette valeur est légèrement supérieure à celles des poudres de sorbitol de l'art antérieur obtenues par granulation. Ceci est d'autant plus remarquable que par rapport à ces produits antérieurs, le sorbitol pulvérulent conforme à l'invention présente une granulométrie plus fine.

Du point de vue de sa composition chimique, le sorbitol pulvérulent conforme à l'invention est relativement pur, c'est à dire qu'il présente une richesse en sorbitol élevée, généralement supérieure à 95 % et plus particulièrement supérieure à 98 % en poids.

Cette richesse élevée se traduit également par un point de fusion élevé, déterminé par analyse micro-calorimétrique (DSC), compris entre 98 et 99,5°C , plus particulièrement compris entre 98,9 et 99,2 °C qui est parmi les valeurs les plus importantes jamais mesurées pour des sorbitols poudre utilisés en compression directe.

Sans vouloir être lié par une quelconque théorie, on peut penser que les caractéristiques physico-chimiques précédemment citées du sorbitol pulvérulent conforme à l'invention expliquent son excellente aptitude à l'écoulement. Ces caractéristiques concernent en particulier sa richesse en sorbitol, sa granulométrie centrée, sa faible hygroscopicité mais également la forme caractéristique de ses particules. Concernant ce dernier point, l'observation au microscope électronique à balayage à faible grossissement (magnitude 50) révèle que le sorbitol pulvérulent conforme à l'invention est généralement constitué de particules de formes variables présentant peu d'arêtes, en majorité composées de micro-particules agglomérées entre elles. A plus fort grossissement (magnitude 1500), lesdites particules présentent à leur surface de nombreuses fines aiguilles de sorbitol γ sans orientation particulière, ce qui est caractéristique d'une abondante recristallisation en surface, et de larges zones ayant un aspect plus fondu dont l'importance relative peut être attribuée aux variantes des procédés mis en oeuvre pour leur fabrication.

Ces dernières caractéristiques peuvent contribuer à expliquer l'excellente aptitude à l'écoulement du sorbitol pulvérulent conforme à l'invention et permettent en tous les cas de le différencier également des sorbitols pulvérulents du commerce.

A la connaissance de la société Demanderesse, ces formes particulières n'ont jamais été décrites pour un sorbitol pulvérulent. Le sorbitol pulvérulent conforme à l'invention se différencie alors aisément d'un sorbitol pulvérulent obtenu par simple atomisation, composé de particules essentiellement sphériques, ou d'un sorbitol obtenu par extrusion, comportant des particules anguleuses en forme d'amas de fines aiguilles orientées dans la même direction.

Le sorbitol pulvérulent conforme à l'invention est susceptible d'être obtenu en procédant à une étape de granulation d'un sorbitol poudre par voie humide à l'aide d'un liant, puis à une étape de maturation par séchage du sorbitol granulé ainsi obtenu. Pour obtenir un sorbitol pulvérulent conforme à l'invention possédant les caractéristiques fonctionnelles énoncées, la société Demanderesse a constaté qu'il convenait de choisir comme sorbitol de départ un sorbitol poudre, pouvant être obtenu par dragéification, par atomisation, par extrusion ou par cristallisation dans l'eau ou dans un autre solvant comme l'alcool. La granulométrie dudit sorbitol poudre de départ ne constitue pas en soi un facteur limitant pour produire un sorbitol pulvérulent conforme à l'invention.

Le liant est quant à lui constitué d'eau ou d'un sirop de sorbitol d'une matière sèche au plus égale à 100 %, de préférence compris entre 10 et 80 %.

De manière surprenante et inattendue, la société Demanderesse a constaté que la granulation d'un sorbitol poudre par voie humide à l'aide d'un liant permet de préparer avec un haut rendement un produit conforme a l'invention sur le plan de son hygroscopicité, de sa surface spécifique, de sa densité, de sa granulométrie et de son aptitude à l'écoulement. En effet, les procédés décrits antérieurement ne permettent pas d'obtenir l'ensemble des caractéristiques souhaitées.

Pour procéder à la granulation, on peut employer par exemple un mélangeur-granulateur continu de type FLEXOMIX vertical commercialisé par la société HOSOKAWA SCHUGI, ou de type CB horizontal commercialisé par la société LÖDIGE dans lequel on introduit, via un doseur pondéral, le sorbitol poudre de départ à granuler en continu et via un doseur volumétrique, le liant (de l'eau ou la solution de sorbitol) en continu. La granulation peut également s'effectuer dans une tour d'atomisation, ou dans un granulateur à lit fluidisé.

Dans un premier mode préférentiel de réalisation du procédé de préparation d'un sorbitol pulvérulent conforme à l'invention, on choisit d'utiliser un mélangeur-granulateur continu de type FLEXOMIX vertical HOSOKAWA SCHUGI. La poudre de sorbitol de départ et le liant sont très intimement mélangés dans le mélangeur-granulateur équipé d'un axe avec couteaux disposés en pales, et d'un système de pulvérisation de liquides par buses d'injection.

Dans un mode préférentiel du procédé, la bonne dispersion des constituants et l'agglomération des particules de la poudre de sorbitol de départ sont réalisées par agitation à grande vitesse, i.e. d'une valeur au moins égale à 1500 tpm, de préférence au moins égale à 3000 tpm. A la sortie du mélangeur-granulateur, les granulés formés sont déchargés en continu sur un séchoir. Le déchargement se fait préférentiellement par gravité dans le cas dudit granulateur vertical, et par poussée, via l'axe des couteaux rotatifs, si le granulateur horizontal est utilisé.

Cette deuxième étape de séchage en sortie du mélangeur-granulateur permet d'éliminer l'eau provenant du liant et de cristalliser la matière sèche provenant du liant, dans le cas où une solution de sorbitol a été mise en oeuvre, de manière à ce que la cristallisation se produise après l'étape préalable de granulation. Le séchoir peut être par exemple un séchoir à lit fluidisé ou un tambour maturateur rotatif. Le sorbitol pulvérulent conforme à l'invention est obtenu après refroidissement et éventuellement tamisage. Dans ce cas, les fines particules peuvent être directement recyclées en tête de granulation, et les grosses particules être broyées et recyclées en tête de tamisage ou en tête de granulation.

Dans un second mode de réalisation préférentiel du procédé de préparation d'un sorbitol pulvérulent conforme à l'invention, on choisit de réaliser la granulation du sorbitol poudre par voie humide en tour d'atomisation. On introduit alors du sorbitol cristallisé dans ladite tour d'atomisation et on ajoute comme liant de l'eau ou un sirop de sorbitol d'une matière sèche au plus égale à 100%, de préférence comprise entre 10 et 80% en poids.

On choisit d'alimenter une tour d'atomisation MSD (Multi-Stage Dryer) de capacité d'évaporation d'eau de l'ordre de 350 kg/h avec du sorbitol poudre à un débit compris entre 400 et 600 kg/h, la granulation se faisant avec de l'eau comme liant, comme il sera exemplifié ci-après.

Au vu des points de fusion des différentes formes cristallines du sorbitol, la Société Demanderesse a trouvé qu'il fallait gérer avec rigueur le suivi des températures de fonctionnement de la tour d'atomisation.

On choisit donc avantageusement de régler la température de l'air d'alimentation à une valeur comprise entre 140 et 145°C, la température des buées à une valeur comprise entre 70 et 75°C et la température du lit statique à une valeur comprise entre 70 ec 80°C.

Le sorbitol pulvérulent conforme à l'invention peut avantageusement être employé, en raison de la qualité de ses propriétés fonctionnelles mentionnées plus haut, dans l'application «comprimés à sucer».

Les comprimés préparés à partir dudit sorbitol présentent en effet, en plus d'une compressibilité élevée qui se traduit par une dureté élevée pour une faible densité, une texture lisse en bouche. Cette dernière propriété organoleptique est particulièrement appréciée pour la fabrication de tablettes ou de comprimés, car un caractère «râpeux» pour des comprimés est considéré comme un caractère non hédonistique par les spécialistes dans ce domaine.

D'autres caractéristiques et avantages de l'invention apparaîtront à la lecture des exemples qui suivent. Ils ne sont toutefois donnés ici qu'à titre illustratif et non limitatif.

### EXEMPLE 1

On alimente un mélangeur-granulateur FLEXOMIX vertical SCHUGI en continu via un doseur poudre, à un débit de 500 kg/h, avec un sorbitol poudre fabriqué par dragéification.

D'autre part, on alimente le mélangeur-granulateur en continu avec de l'eau à 60°C et à un débit de 40 l/h, via une buse de pulvérisation. L'axe rotatif à couteaux est préalablement réglé à la vitesse de 3000 tpm. La poudre granulée humide en sortie du mélangeur-granulateur tombe en continu, par gravité, dans un séchoir à lit fluidisé SCHU-Gl à deux compartiments.

Dans le premier compartiment, le produit granulé est séché par de l'air à 120°C, puis il est refroidi par de l'air à 20°C dans le deuxième compartiment. Le produit granulé, séché et refroidi est ensuite tamisé en continu sur un tamis rotatif équipé de deux toiles métalliques de 120 et 600 µm. Le sorbitol poudre de départ A et le sorbitol pulvérulent B ainsi obtenu conforme à l'invention présentent les caractéristiques rassemblées dans le tableau I suivant.

**Tableau I**

| **Paramètres** | **A** | **B** |
|---|---|---|
| Richesse en sorbitol (% en poids) | 98,5 | 98,5 |
| Teneur en eau (%) | 0,5 | 0,4 |
| Température de fusion (selon DSC ; °C) | 98,5 | 99,2 |
| Chaleur de fusion (selon DSC ; J/g) | 168 | 171 |
| Hygroscopicité (% à 60% ERH) | 1,7 | 1,3 |
| Surface spécifique (m'/g) | 0,8 | 2,4 |
| Densité apparente (g/ml) | 0,61 | 0,5 |
| Diamètre moyen (µm) | 100 | 155 |
| Note d'écoulement (valeur/100) | 65 | 75 |
| Compressibilité (N) | 60 | 120 |
| Friabilité (%) | 18 | 26 |

### EXEMPLE 2

On alimente un mélangeur-granulateur FLEXOMIX vertical SCHUGI en continu via un doseur poudre, avec le sorbitol poudre de départ A, et dans les mêmes conditions que dans l'exemple 1, mais on alimente ledit mélangeur-granulateur avec une solution de sorbitol à 70% de matière sèche comme liant, à un débit de 40 l/h et une température de 60°C via une buse de pulvérisation.

Les sorbitol pulvérulents C et D conformes à l'invention, obtenus respectivement avec une température de l'air de chauffage de 120°C et de 75°C, présentent les caractéristiques rassemblées dans le tableau II suivant.

**Tableau II**

| **Paramètres** | **C** | **D** |
|---|---|---|
| Richesse en sorbitol (% en poids) | 98,5 | 98,5 |
| Teneur en eau (%) | 0,45 | 0,55 |
| Température de fusion (selon DSC ; °C) | 98,9 | 99,2 |
| Chaleur de fusion (selon DSC ; J/g) | 172 | 175 |
| Hygroscopicité (% à 60% ERH) | 1,2 | 1,4 |
| Surface spécifique (m²/g) | 2,75 | 2,2 |
| Densité apparente (g/ml) | 0,47 | 0,42 |
| Diamètre moyen (µm) | 192 | 193 |
| Note d'écoulement (valeur/100) | 75 | 74 |
| Compressibilité (N) | 120 | 130 |
| Friabilité (%) | 25 | 22 |

### EXEMPLE 3

On conduit le procédé de la même manière que dans l'exemple 1, mais avec un sorbitol poudre de départ de granulométrie plus fine. Le sorbitol poudre E de départ, et le sorbitol pulvérulent F conforme à l'invention présentent les caractéristiques rassemblées dans le tableau III suivant.

**Tableau III**

| **Paramètres** | **E** | **F** |
|---|---|---|
| Richesse en sorbitol (% en poids) | 98 | 98 |
| Teneur en eau (%) | 0,5 | 0,3 |
| Température de fusion (selon DSC ; °C) | 97,5 | 99.2 |
| Chaleur de fusion (selon DSC ; J/g) | 168 | 169 |
| Hygroscopicité (% à 60% ERH) | 1,9 | 1,1 |
| Surface spécifique (m²/g) | 0,9 | 3,25 |
| Densité apparence (g/ml) | 0,5 | 0.48 |
| Diamètre moyen (µm) | 60 | 193 |
| Note d'écoulement (valeur/100) | 60 | 73.5 |
| Compressibilité (N) | 65 | 122 |
| Friabilité | nd | 32 |

### EXEMPLE 4

On conduit le procédé avec le même sorbitol poudre de départ E qui a été mis en oeuvre dans l'exemple 3. Une tour d'atomisation MSD de 350 kg/h de capacité d'évaporation est alimentée avec le sorbitol poudre E à raison de 410 kg/h.

La granulation à l'eau est réalisée en pulvérisant de l'eau à raison de 80 l/h par une buse à 45 bars de pression.

L'air de séchage entre à 136°C et ressort à 78°C et la température des buées est déterminée à 142°C, le lit statique en bas de la tour étant refroidi par de l'air à 70°C.

En sortie de la tour d'atomisation, le produit passe sur un lit fluide vibré où il est refroidi par de l'air en 3 zones de température fixées respectivement à 35°C, 20°C et 20°C.

Le produit G présente les caractéristiques rassemblées dans le tableau IV suivant :

**Tableau IV**

| **Paramètres** | **E** | **F** |
|---|---|---|
| R_chesse en sorbitol (% en poids) | 98 | 98 |
| Teneur en eau (%) | 0,5 | 0,25 |
| Température de fusion (selon DSC ; °C) | 97,5 | 99 |
| Chaleur de fusion (selon DSC ; J/g) | 168 | 169 |
| Hygroscopicite (% à 60% ERH) | 1.9 | 1 |
| Surface spécifique (m/g) | 0,9 | 3,25 |
| Densité apparente (g/ml) | 0,5 | 0,47 |
| Diametre moyen (µm) | 60 | 300 |
| Note d'écoulement (valeur 100) | 60 | 80 |
| Compressibilité (N) | 65 | 135 |
| Friabilite | nd | 30 |

### EXEMPLE 5

Des produits conformes à l'invention préparés en appliquant les procédés décrits dans les exemples 1 à 4 sont comparés, dans le tableau V suivant, à des sorbitols pulvérulents connus par ailleurs (cf, par example, FR 2622190 et GB 2046743).

**Tableau V**

| | | Produits comparatifs | | |
|---|---|---|---|---|
| | Produits conformes à l'invention | NEOSORB P60W ROQUETTE | SORBITOL -L MERCK | SORBITOL décrit dans FR 2 622 190 |
| Richesse en sorbitol (% en poids) | 98 - 100 | 94,5 - 97,8 | 97,7 - 98 | 97,2 - 98,2 |
| Teneur en eau (%) | 0,35 - 0,45 | 0,5 | 0,6 - 0,8 | < 1 |
| Température de fusion (DSC, °C) | 98,9 -99,2 | 94,5 - 98 | 94,8 - 95 | 94,5 - 96 |
| Chaleur de fusion (DSC ; J/gl | 165 - 175 | 165 | 155 - 165 | - |
| Hygroscopicité (% à 60% ERH) | 1,1 à 1,4 | 1,53 | 2,40 | - |
| Surface spécifique (m²/g) | 2,2 - 3,25 | < 1 | 1,55 | 1-1,2 |
| Diamètre moyen (µm) | 150 - 250 | 240 - 340 | 270 - 300 | 280 - 520 |
| Densité apparente (g/ml) | 0,4 - 0,6 | 0,4 - 0,65 | 0,45 | 0,54 - 0,6 |
| Note d'écoulement (Valeur / 100) | 70 - 80 | 75 | 78 | - |
| Compressibilité (N) | 120 - 130 | 40 | 150 | 100 |
| Friabilité (%) | 20 - 35 | 20 | 50 | 45 |

Les sorbitols pulvérulents conformes à l'invention possèdent tous, contrairement aux produits de l'art antérieur, d'excellentes propriétés fonctionnelles, les rendant aptes à être utilisés sans inconvénient comme excipients et supports d'additifs non hygroscopiques, en particulier dans les industries alimentaire et pharmaceutique.

### EXEMPLE 6.

On évalue par analyse sensorielle l'effet du sorbitol pulvérulent conforme à l'invention relativement au sorbitol de l'art antérieur sur la texture en bouche de comprimés préparés à partir desdits sorbitol. Ce test organoleptique D est effectué de la manière suivante.

Pour chaque sorbitol pulvérulent, en l'occurrence ici un sorbitol commercialisé par la société MERCK sous le nom SORBITOL grade L , et un sorbitol conforme à l'invention préparé selon l'exemple 1, on prépare une série de comprimés convexes, d'un diamètre de 13 mm, obtenus sur presse alternative FROGERAIS AM, après mélange extemporané avec 0,7 % de stéarate de magnésium.

Chaque série est alors évaluée «en aveugle» par un jury expert de 10 personnes. Ces dernières ont alors à se prononcer sur le caractère «lisse en bouche» desdits comprimés sucés. La synthèse des évaluations obtenues pour chaque type de comprimé fabriqué à partir du sorbitol conforme à l'invention permet de les comparer à ceux préparés avec le sorbitol de l'art antérieur, et ce, selon la notation suivante :
- notation «- - -» : aucune perception texture «lisse en bouche» , au contraire, caractère râpeux prononcé,
- notation «+ +» : texture lisse en bouche nettement perceptible, mais avec une légère sensation de caractère râpeux,
- notation «+ + +» : texture lisse en bouche perçue, sans aucune sensation de caractère râpeux.

Les résultats de ces tests relatifs au caractère hédonique des comprimés préparés à partir des sorbitols pulvérulents sont repris dans le tableau VI ci-après, en fonction des paramètres de poids, de densité et de dureté des comprimés utilisés.

**Tableau VI**

| **Type de sorbitol utilisé** | **Poids (mg) des comprimés** | **Densité (*mg*/*ml*) des comprimés** | **Dureté (N) des comprimés** | **Surface lisse en bouche** |
|---|---|---|---|---|
| SORBITOL L MERCK | 652 | 1,154 | > 196 | --- |
| Sorbitol pulvérulent conforme à l'invention | 651 | 1,152 | 161 | ++ |
| | 686 | 1,214 | > 196 | +++ |

On constate que les comprimés fabriqués avec le sorbitol pulvérulent conforme à l'invention diffèrent des comprimés fabriqués avec l'autre sorbitol, par le fait qu'ils présentent simultanément une compressibilité élevée (dureté élevée pour une faible densité) et une texture lisse en bouche.

## Revendications

1. Sorbitol pulvérulent **caractérisé en ce qu'**il présente :
- une valeur d'hygroscopicité, déterminée selon un test A, inférieure à 2 %, de préférence inférieure à 1,7 %, le test A consistant à établir la courbe d'isotherme de sorption d'eau à 20°C exprimant le pourcentage de reprise en eau d'un produit pulvérulent préalablement déshydraté que l'on place dans une atmosphère de teneur en humidité relative variable et à une température de 20°C, la détermination de l'hygroscopicité du produit pulvérulent étant alors le pourcentage de reprise en eau, à 60 % d'humidité relative à l'équilibre (ou 60 % E.R.H. pour Equilibrium Relative Humidity),
- une surface spécifique, déterminée selon la méthode BET, au moins égale à 2 m²/g, de préférence au moins égale à 2,2 m²/g.

2. Sorbitol pulvérulent selon la revendication 1, **caractérisé en ce qu'**il présente une valeur d'hygroscopicité comprise entre 0,5 et 1,6 %, de préférence comprise entre 0,9 et 1,4 %.

3. Sorbitol pulvérulent selon l'une quelconque des revendications 1 à 2, **caractérisé en ce qu'**il présente :
- une densité apparente, déterminée selon la méthode HOSOKAWA, comprise entre 0,35 et 0,65 g/ml, de préférence comprise entre 0,4 et 0,6 g/ml,
- une compressibilité, déterminée selon un test B, comprise entre 100 et 150 N, et plus particulièrement comprise entre 120 et 140 N, le test B consistant à mesurer la force exprimée en Newton, qui est représentative de la compressibilité du sorbitol pulvérulent étudié, cette force traduisant donc ici la résistance à l'écrasement d'un comprimé qui est cylindrique à faces convexes (rayon de courbure de 14 mm), d'un diamètre de 13 mm, d'une épaisseur de 6 mm et d'un poids de 0,647 g, soit d'une masse volumique apparente de 1,1 g/ml,
- une friabilité, déterminée selon un test C, comprise entre 10 et 50 %, de préférence entre 20 et 40 %.

4. Sorbitol pulvérulent selon l'une quelconque des revendications 1 à 3, **caractérisé en ce qu'**il présente :
- un diamètre moyen compris entre 150 et 250µm,
- une note d'écoulement d'au moins 70, de préférence comprise entre 70 et 90, et plus préférentiellement comprise entre 70 et 80.

5. Sorbitol pulvérulent selon l'une quelconque des revendications 1 à 4, **caractérisé en ce qu'**il présente :
- une richesse en sorbitol supérieure à 95 %, de préférence supérieure à 98 %,
- un point de fusion compris entre 98 et 99,5 °C, de préférence compris entre 98,9 et 99,2°C,
- une organisation cristalline dans laquelle d'abondantes aiguilles fines de sorbitol gamma sans orientation particulière se trouvent à la surface de particules de formes variables, associées à des zones d'aspect plus fondu.

6. Procédé de préparation d'un sorbitol pulvérulent selon l'une quelconque des revendications 1 à 5, **caractérisé en ce qu'**il comprend une étape de granulation d'un sorbitol poudre par voie humide à l'aide d'un liant et une étape de maturation, par séchage, du sorbitol granulé ainsi obtenu.

7. Procédé de préparation selon la revendication 6, **caractérisé en ce que** l'étape de granulation est réalisée dans un mélangeur-granulateur continu.

8. Procédé de préparation selon la revendication 6, **caractérisé en ce que** l'étape de granulation est réalisée dans une tour d'atomisation.

9. Utilisation de sorbitol pulvérulent selon l'une quelconque des revendications 1 à 5 en tant qu'agent édulcorant, agent de texture, excipient ou support d'additif dans des compositions destinées en particulier aux domaines alimentaire et pharmaceutique.

10. Utilisation de sorbitol pulvérulent selon l'une quelconque des revendications 1 à 5, pour la préparation de comprimés présentant, selon un test D consistant à préparer une série de comprimés convexes, d'un diamètre de 13 mm, obtenus sur une presse alternative FROGERAIS AM, après mélange extemporané avec 0,7 % de stéarate de magnésium, une texture lisse en bouche, c-à-d, aucune sensation de caractère râpeux.

## Patentansprüche

1. Pulverförmiger Sorbit, **dadurch gekennzeichnet, daß** er folgende Daten aufweist
- einen in einem Test A bestimmten Hygroskopizitätswert unter 2%, vorzugsweise unter 1,7%, wobei der Test A darin besteht, daß die Wassersorptions-Isotherme bei 20°C erstellt wird, die den Prozentsatz der Wasseraufnahme eines zuvor dehydratisierten pulverförmigen Produkts ausdrückt, das man in eine Atmosphäre mit veränderlichem Gehalt an relativer Feuchtigkeit und mit einer Temperatur von 20°C bringt, wobei die Bestimmung der Hygroskopizität des pulverförmigen Produkts hierbei der Prozentsatz der Wasseraufnahme bei 60% relativer Feuchtigkeit im Gleichgewicht (oder 60% E.R.H. für Equilibrium Relative Humidity) ist,
- eine nach der Methode BET bestimmte spezifische Oberfläche von mindestens gleich 2 m²/g, vorzugsweise mindestens gleich 2,2 m²/g.

2. Pulverförmiger Sorbit nach Anspruch 1, **dadurch gekennzeichnet, daß** er einen Hygroskopizitätswert zwischen 0,5 und 1,6 %, vorzugsweise zwischen 0,9 und 1,4 %, aufweist.

3. Pulverförmiger Sorbit nach einem der Ansprüche 1 bis 2, **dadurch gekennzeichnet, daß** er folgende Daten aufweist :
- eine nach der Methode HOSOKAWA bestimmte scheinbare Dichte zwischen 0,35 und 0,65 g/ml, vorzugsweise zwischen 0,4 und 0,6 g/ml,
- eine in einem Test B bestimmte Komprimierbarkeit zwischen 100 und 150 N und insbesondere zwischen 120 und 140 N, wobei der Test B darin besteht, daß man die in Newton ausgedrückte Kraft mißt, die ein Ausdruck für die Komprimierbarkeit des untersuchten pulverförmigen Sorbits ist, wobei diese Kraft hier also die Druckfestigkeit einer zylindrischen Tablette mit konvexen Seiten (Krümmungsradius 14 mm) mit einem Durchmesser von 13 mm, einer Dicke von 6 mm und einem Gewicht von 0,647 g, d.h. einer scheinbaren Volumenmasse von 1,1 g/ml, darstellt,
- eine in einem Test C bestimmte Mürbheit zwischen 10 und 50 %, vorzugsweise zwischen 20 und 40 %.

4. Pulverförmiger Sorbit nach einem der Ansprüche 1 bis 3, **dadurch gekennzeichnet, daß** er folgende Daten aufweist:
- einen mittleren Durchmesser zwischen 150 und 250 µm,
- eine Fließzahl von mindestens 70, vorzugsweise zwischen 70 und 90 und noch bevorzugter zwischen 70 und 80.

5. Pulverförmiger Sorbit nach einem der Ansprüche 1 bis 4, **dadurch gekennzeichnet, daß** er folgende Daten auf- weist:
- einen Gehalt an Sorbit über 95 %, vorzugsweise über 98 %,
- einen Schmelzpunkt zwischen 98 und 99,5 °C, vorzugsweise zwischen 98,9 und 99,2 °C,
- einen Kristallaufbau, bei dem reichliche dünne Nadeln von Sorbit γ ohne besondere Ausrichtung sich an der Oberfläche von Teilchen mit veränderlichen Formen befinden, denen Zonen mit mehr geschmolzenem Aus- sehen zugeordnet sind.

6. Verfahren zur Herstellung von pulverförmigem Sorbit nach einem der Ansprüche 1 bis 5, **dadurch gekennzeichnet, daß** es die Schritte der nassen Granulierung von pulverförmigem Sorbit mit Hilfe eines Bindemittels und einen Schritt der Reifung des auf diese Weise erhaltenen granulierten Sorbits durch Trocknung umfaßt.

7. Herstellungsverfahren nach Anspruch 6, **dadurch gekennzeichnet, daß** der Schritt der Granulierung in einer kontinuierlichen Misch- und Granuliervorrichtung durchgeführt wird.

8. Herstellungsverfahren nach Anspruch 6, **dadurch gekennzeichnet, daß** der Schritt der Granulierung in einem Zerstäubungsturm durchgeführt wird.

9. Verwendung von pulverförmigem Sorbit nach einem der Ansprüche 1 bis 5 als Süßungsmittel, Texturmittel, Zusatzträger oder -grundlage in insbesondere für den Nahrungsmittel- und pharmazeutischen Bereich bestimmten Zusammensetzungen.

10. Verwendung von pulverförmigem Sorbit nach einem der Ansprüche 1 bis 5, für die Herstellung von Tabletten, die in einem Test D, der darin besteht, daß eine Reihe von konvexen Tabletten mit einem Durchmesser von 13 mm hergestellt werden, die auf einer sich hin- und herbewegenden Presse FROGERAIS AM nach Direktmischung mit 0,7 % Magnesiumstearat erhalten wurden, eine glatte Textur im Mund, d.h. keine Wahrnehmung eines rauhen Charakters, aufweisen.

## Claims

1. Pulverulent sorbitol, **characterized in that** it exhibits:
- a hygroscopicity value, determined according to a test A, of less than 2%, preferably of less than 1.7%, the test consisting in drawing up the isothermal curve for water sorption at 20°C which expresses the percentage of water uptake of a pulverulent product, dehydrated beforehand, which is placed in an atmosphere of variable relative humidity and at a temperature of 20°C, the determination of the hygroscopicity of the pulverulent product then being the percentage of water uptake at 60% equilibrium relative humidity (or 60% E.R.H.),
- a specific surface, determined according to the BET method, at least equal to 2 m2/g, preferably at least equal to 2.2 m2/g.

2. Pulverulent sorbitol according to Claim 1, **characterized in that** it exhibits a hygroscopicity value of between 0.5 and 1.6%, preferably of between 0.9 and 1.4%.

3. Pulverulent sorbitol according to any one of Claims 1 to 2, **characterized in that** it exhibits:
- a bulk density, determined according to the Hosokawa method, of between 0.35 and 0.65 g/ml, preferably of between 0.4 and 0.6 g/ml,
- a compressibility, determined according to a test B, of between 100 and 150 N, and more particularly of between 120 and 140 N, the test B consisting in measuring the force, expressed in newtons, which is representative of the compressibility of the pulverulent sorbitol studied, this force therefore expressing, in this instance, the resistance to crushing of a tablet which is cylindrical with convex faces (radius of curvature of 14 mm), with a diameter of 13 mm, with a thickness of 6 mm and with a weight of 0.647 g, i.e. with a bulk density of 1.1 g/ml,
- a friability, determined according to a test C, of between 10 and 50%, preferably between 20 and 40%.

4. Pulverulent sorbitol according to any one of Claims 1 to 3, **characterized in that** it exhibits:
- a mean diameter of between 150 and 250 µm,
- a flow grade of at least 70, preferably of between 70 and 90, and more preferably of between 70 and 80.

5. Pulverulent sorbitol according to any one of Claims 1 to 4, **characterized in that** it exhibits:
- a sorbitol content of greater than 95%, preferably of greater than 98%,
- a melting point of between 98 and 99.5°C, preferably of between 98.9 and 99.2°C,
- a crystalline organization in which copious fine needles of γ sorbitol without specific orientation are found at the surface of particles of variable shapes, in combination with regions with a more molten appearance.

6. Process for the preparation of a pulverulent sorbitol according to any one of Claims 1 to 5, **characterized in that** it comprises a stage of granulation of a sorbitol powder by the wet route using a binder and a stage of maturation, by drying, of the granulated sorbitol thus obtained.

7. Preparation process according to Claim 6, **characterized in that** the granulation stage is carried out in a continuous mixer-granulator.

8. Preparation process according to Claim 6, **characterized in that** the granulation stage is carried out in an atom- ization tower.

9. Use of a pulverulent sorbitol according to any one of claims 1 to 5, as a sweetening agent, texturizing agent, excipient or additive support in compositions intended in particular for the food and pharmaceutical fields.

10. Use of a pulverulent sorbitol according to any one of claims 1 to 5, for the preparation of tablets exhibiting, according to a test D consisting in preparing a series of convex tablets, with a diameter of 13 mm, obtained on a Frogerais AM reciprocating press, after mixing at the time of tableting with 0.7% of magnesium stearate, a "smooth in the mouth" texture, i.e. without any rough character feeling.
